(19) Europäisches Patentamt
European Patent Office
Office européen des brevets

(11) Veröffentlichungsnummer: **0 115 324 B1**

# (12) EUROPÄISCHE PATENTSCHRIFT

(45) Veröffentlichungstag der Patentschrift: **26.10.88**

(51) Int. Cl.⁴: **C 07 D 239/46**

(21) Anmeldenummer: **84100717.2**

(22) Anmeldetag: **24.01.84**

(54) **Verfahren zur Herstellung von 2,4,5,6-Tetraaminopyrimidin-Sulfat.**

(30) Priorität: **28.01.83 CH 481/83**

(43) Veröffentlichungstag der Anmeldung: **08.08.84 Patentblatt 84/32**

(45) Bekanntmachung des Hinweises auf die Patenterteilung: **26.10.88 Patentblatt 88/43**

(84) Benannte Vertragsstaaten: **BE CH DE FR GB IT LI LU NL**

(56) Entgegenhaltungen: **GB - A - 763 120**

(73) Patentinhaber: **LONZA AG, Gampel/Wallis (CH)**

(72) Erfinder: **O'Murchu, Colm, Dr., Gebreitenweg 4 B, Visp (Kanton Wallis) (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 324 B1

ACTORUM AG

**Beschreibung**

Die Erfindung betrifft ein Verfahren zur Herstellung von 2,4,5,6-Tetraaminopyrimidin-sulfat aus 2,4,6-Triamino-5-phenylazopyrimidin.

Es ist bekannt, durch Reduktion von 2,4,6-Triamino-5-nitrosopyrimidin 2,4,5,6-Tetraaminopyrimidin herzustellen, welches durch Ansäuren mit $H_2SO_4$ 2,4,5,6-Tetraaminopyrimidin-sulfat liefert. Gemäss Ber. 37 4544 (1904), sowie J. Chem. Soc. Jap. Pure Chem. Sect. 72 866 (1951); Chem. Abstr. 47 5946 (1953) wird Ammoniumsulfid als Reduktionsmittel verwendet. In J. Am. Chem. Soc. 69 1814 (1947) wird 2,4,6-Triamino-5-nitrosopyrimidin mit Natriumdithionit reduziert und als Produkt 2,4,5,6-Tetraaminopyrimidin-bisulfit in 54% Ausbeute erhalten. Nach der NL-PS 77 12 155 kann die Reaktion auch katalytisch durchgeführt werden, z. B. mit Raney-Nickel oder mit Nickel-Salzen und Natriumborhydrid. Ferner wurden Natriumdithionit oder Ni/-Hydrazin (US-PS 4 167 633) und Zn-HCl (US-PS 4 247 693) als Reduktionsmittel verwendet.

Gemäss Chem. and Pharm. Bull. (Tokyo) 7 1 (1959) wird 2,4,5,6-Tetraaminopyrimidin-sulfat durch katalytisches Hydrieren von 4-(2,4,6-Triamino-5-pyrimidinylazo)-benzol-sulfonsäure in wässriger Natriumbicarbonat-Lösung hergestellt.

Dieses Verfahren hat den Nachteil, dass eine grosse Menge Säure für die Neutralisation benötigt wird, was zu einer zu grossen Belastung des Abwassers führt.

In der GB-PS 763 120 ist die Reduktion von 5-p-Chlorphenylazo-6-aminopyrimidinen, die in der 2- und 4-Stellung mit verschiedenen Mono- und Dialkylaminogruppen substituiert sind, mit Raney-Nickel/$H_2$ in Ethanol beschrieben. Die resultierende Mischung wird danach mit Eisessig versetzt und der sich bildende Niederschlag wird filtriert und unter Stickstoff zur Trockene eingedampft. Daran schliesst sich eine Extraktion des getrockneten Niederschlags mit Benzol an, worauf der verbleibende Rückstand wieder in Ethanol gelöst und diese Lösung schliesslich zur Ausfällung des entsprechenden 2,4-disubstituierten 5,6-Diaminopyrimidinsulfats mit konzentrierter Schwefelsäure versetzt wird. Dieses Verfahren beinhaltet offensichtlich sehr viele Stufen und umfasst einen Trocknungsschritt unter einer inerten Atmosphäre.

Aufgabe der vorliegenden Erfindung ist es, ein Verfahren zur Verfügung zu stellen, das die Nachteile der bekannten Verfahren nicht aufweist.

Die Reaktion folgt der allgemeinen Formel

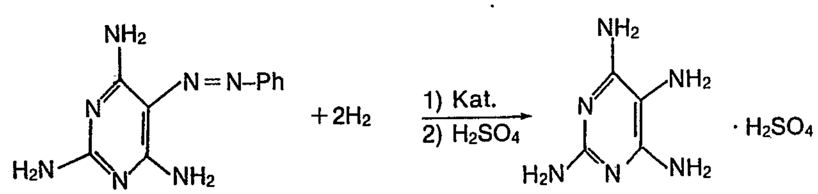


Für die Hydrierung können Wasserstoffüberdrucke von 0 bis 60 bar, vorzugsweise 0 bis 10 bar, angewendet werden. Die Reaktionstemperatur beträgt 50 bis 180 °C, vorzugsweise 80 bis 120 °C.

Die Reaktionsdauer ist von der Reaktionstemperatur abhängig. Zweckmässig beträgt die Reaktionsdauer 1 bis 3 Stunden.

Das Verhältnis Wasser zu Edukt beträgt zweckmässig 3 zu 1 bis 30 zu 1.

Statt in Wasser kann das Edukt auch in Alkohol suspendiert oder in verdünnter Lauge gelöst werden.

Als Katalysator werden die handelsüblichen Kohlenstoff/Palladium-Katalysatoren angewendet, wobei vorzugsweise Katalysatoren mit 5% Palladium auf Kohle eingesetzt werden.

Durch Filtration wird die Reaktionslösung vom Katalysator abgetrennt und das 2,4,5,6-Tetraaminopyrimidin-sulfat wird durch Zugabe von Schwefelsäure ausgefällt. Die Konzentration der Schwefelsäure ist an sich unkritisch, aus technischen Gründen soll sie nicht über 60 Gew.% liegen, bevorzugt werden Konzentrationen von 5 bis 35 Gew.%. Die Menge der Schwefelsäure liegt zweckmässig über einem Äquivalent, bevorzugt bei 1,1 bis 2 Äquivalent. Durch Zentrifugieren oder Filtrieren wird das 2,4,5,6-Tetraaminopyrimidinsulfat abgetrennt und mit Wasser gewaschen. Bei dieser Variante kann das gebildete Anilin in Anilinsulfat umgewandelt werden, welches in der Mutterlauge bleibt.

Eine Variation dieses Verfahrens besteht darin, dass vor der Zugabe von $H_2SO_4$ das Anilin azeotrop mit Wasser abdestilliert wird. Bei der Ausfällung von 2,4,5,6-Tetraaminopyrimidin-sulfat wird dann die entsprechende Menge weniger $H_2SO_4$ benötigt.

Das erfindungsgemäss hergestellte Produkt kann zu Diaminopurin, Methotrexat oder zu anderen Pteridinen umgesetzt werden, oder es findet Verwendung als Entwicklerkomponente für Oxidationshaarfarbstoffe.

Beispiel 1

Eine Suspension von 77 g 5-Phenylazo-2,4,6-triamino-pyrimidin und 2 g Palladium auf Kohle-Katalysator in 500 ml Wasser wurden in einem Autoklaven bei 5 bis 10 bar und 90 bis 115 °C während 1 Stunde hydriert. Das auf 70 °C abgekühlte Reaktionsgemisch wurde unter Luftausschluss vom Katalysator abfiltriert und mit 265 g 25%iger $H_2SO_4$ angesäuert. Das ausgefallene 2,4,5,6-Tetraaminopyrimidin-sulfat wurde abgenutscht und mit Wasser gewaschen. Nach dem Trocknen bei 100 °C im Vacuum erhielt man 76,2 g 2,4,5,6-Tetraaminopyrimidin-sulfat mit einem Gehalt von 97,5% und 1,3% Restfeuchtigkeit.

Beispiel 2

Eine Suspension von 77 g 5-Phenylazo-2,4,6-triamino-pyrimidin und 2 g Palladium auf Kohle-Katalysator in 500 ml Wasser wurden in einem Autoklaven bei 5 bis 10 bar und 90 bis 115 °C während 90 Minuten hydriert. Nach Abtrennen vom Katalysator wurde das Reaktionsgemisch erhitzt, um ein Gemisch aus Anilin und Wasser abzudestillieren. Als kein Anilin mehr destilliert wurde, verdünnte man die Reaktionslösung mit 200 ml Wasser und stellte den pH unter 1, durch Zugabe von 230 g 20%iger $H_2SO_4$. Das ausgefallene 2,4,5,6-Tetraaminopyrimidin-sulfat wurde abgenutscht, mit Wasser gewaschen und getrocknet.

Man erhielt 77,2 g 2,4,5,6-Tetraaminopyrimidin-sulfat mit einem Gehalt von 97,5% und 0,5% Restfeuchtigkeit.

**Patentansprüche**

1. Verfahren zur Herstellung von 2,4,5,6-Tetraaminopyrimidinsulfat aus 2,4,6-Triamino-5-phenylazopyrimidin, dadurch gekennzeichnet, dass 2,4,6-Triamino-5-phenylazopyrimidin, in Wasser oder Alkohol suspendiert oder in verdünnter Lauge gelöst, in Gegenwart eines Katalysators, enthaltend Palladium auf Kohle, bei 50 bis 180 °C und 0 bis 60 bar Überdruck hydriert und durch Zugabe von Schwefelsäure ausgefällt wird.

2. Verfahren nach Anspruch 1, dadurch gekennzeichnet, dass die Hydrierung bei 0 bis 10 bar Überdruck durchgeführt wird.

3. Verfahren nach einem oder mehreren der Ansprüche 1 bis 2, dadurch gekennzeichnet, dass die Reaktionstemperatur 80 bis 120 °C beträgt.

4. Verfahren nach einem oder mehreren der Ansprüche 1 bis 3, dadurch gekennzeichnet, dass die Reaktionszeit 1 bis 3 Stunden beträgt.

5. Verfahren nach einem oder mehreren der Ansprüche 1 bis 4, dadurch gekennzeichnet, dass vor der Zugabe von Schwefelsäure das gebildete Anilin durch azeotrope Destillation mit Wasser entfernt wird.

**Claims**

1. Process for the production of 2,4,5,6-tetra-amino-pyrimidine sulfate from 2,4,6-triamino-5-phenylazopyrimidine, characterized in that 2,4,6-triamino-5-phenylazopyrimidine, suspended in water or alcohol or dissolved in a diluted base, is hydrogenated in the presence of a catalyst containing palladium on carbon at 50 to 180 °C and an excess pressure of 0 to 60 bar and precipitated by the addition of sulfuric acid.

2. Process according to claim 1, characterized in that the hydrogenation is carried out at 0 to 10 bar excess pressure.

3. Process according to one or more of claims 1 to 2, characterized in that the reaction temperature is 80 to 120 °C.

4. Process according to one or more of claims 1 to 3, characterized in that the reaction period is 1 to 3 hours.

5. Process according to one or more of claims 1 to 4, characterized in that before adding the sulfuric acid the formed aniline is removed by azeotropic distillation with water.

**Revendications**

1. Procédé pour la préparation de sulfate de 2,4,5,6-tétraaminopyrimidine à partir de 2,4,6-triamino-5-phénylazopyrimidine, caractérisé en ce qu'on met de la 2,4,6-triamino-5-phénylazopyrimidine en suspension dans de l'eau ou de l'alcool ou on la dissout dans une lessive diluée, on hydrogène en présence d'un catalyseur contenant du palladium sur charbon, à 50 à 180 °C et sous 0 à 60 bars de surpression et on fait précipiter par addition d'acide sulfurique.

2. Procédé selon la revendication 1, caractérisé en ce que l'hydrogénation est effectuée sous 0 à 10 bars de surpression.

3. Procédé selon l'une ou plusieurs des revendications 1 à 2, caractérisé en ce que la température de réaction est de 80 à 120 °C.

4. Procédé selon l'une ou plusieurs des revendications 1 à 3, caractérisé en ce que la durée de réaction est de 1 à 3 heures.

5. Procédé selon l'une ou plusieurs des revendications 1 à 4, caractérisé en ce que, avant l'addition de l'acide sulfurique, on élimine l'aniline formée par distillation azéotropique avec de l'eau.